# EUROPÄISCHE PATENTANMELDUNG

(11) **EP 3 772 379 A1**
(43) Veröffentlichungstag der Anmeldung: **10.02.2021**
(21) Anmeldenummer: 19190871.4
(22) Anmeldetag: 08.08.2019
(51) Int. Cl.: B05B 11/00, A45D 34/04, G01F 11/02

(54) **FLÜSSIGKEITSSPENDER**

(71) Anmelder: Aptar Radolfzell GmbH, 78315 Radolfzell (DE)
(72) Erfinder: Bruder, Thomas, 78467 Konstanz (DE); Greiner-Perth, Jürgen, 78244 Gottmadingen (DE); Ritsche, Stefan, 78253 Eigeltingen (DE)
(74) Vertreter: Patentanwaltskanzlei Cartagena

(57) **Zusammenfassung**

Die Erfindung betrifft einen Flüssigkeitsspender (10) zum dosierten Austrag von Flüssigkeit. Der erfindungsgemäße Flüssigkeitsspender (10) weist einen Flüssigkeitsspeicher (20) zur Lagerung der Flüssigkeit vor dem Austrag auf. Er weist weiterhin einen Dosierkopf (40) mit einer Dosierkammer (42) zur Aufnahme einer Charge der Flüssigkeit auf, der gegenüber dem Flüssigkeitsspeicher (20) verlagerbar ist. Es ist eine Pumpeinrichtung (30) vorgesehen, die zwischen dem Flüssigkeitsspeicher (20) und der Dosierkammer (42) angeordnet ist, so dass eine Pumpkammer (32) der Pumpeinrichtung (30) durch Verlagerung des Dosierkopfes (40) in einer Pumprichtung (2) Flüssigkeit in die Dosierkammer (42) fördert.

Es wird vorgeschlagen, dass der Austrag der Flüssigkeit aus der Dosierkammer durch Kontakt des Dosierkopfes mit einer Oberfläche gesteuert wird, wobei hierfür verschiedene Varianten vorgeschlagen werden. So verfügt gemäß einer ersten Variante der Flüssigkeitsspender (10) über eine die Dosierkammer (42) begrenzende Applikatorwandung (80) am Dosierkopf (40), die für den unmittelbaren Körperkontakt vorgesehen ist und die von einer Austragöffnung (82) oder einer Mehrzahl von Austragöffnungen durchdrungen ist, wobei die Applikatorwandung (80) gegenüber angrenzenden Wandungen (84) der Dosierkammer (42) als Ganzes oder durch Verformung in einer Verlagerungsrichtung (6) verlagerbar ist.

## Beschreibung

### ANWENDUNGSGEBIET UND STAND DER TECHNIK

Die Erfindung betrifft einen Flüssigkeitsspender zum dosierten Austrag von Flüssigkeit. Insbesondere handelt es sich hierbei um einen Flüssigkeitsspender zum Austrag von kosmetischen oder pharmazeutischen Flüssigkeiten, beispielsweise von eher hochviskosen Cremes, die auf der Haut aufgetragen werden, oder von eher niederviskosen Flüssigkeiten wie Haarwuchstinkturen, die auf der Kopfhaut aufgetragen werden.

Es besteht der Bedarf nach Flüssigkeitsspendern, die es gestatten, auf bequeme Art und Weise eine vordefinierte Menge der Flüssigkeit auf einer Oberfläche, insbesondere eine Hautoberfläche, aufzutragen.

Aus der DE 102014216744 A1 ist ein Mischspender bekannt, der es gestattet, aus zwei Flüssigkeitsspeichern Flüssigkeit in eine Mischkammer zu fördern, aus der die gemischte Flüssigkeit anschließend weitergefördert werden kann.

### AUFGABE UND LÖSUNG

Ein erfindungsgemäßer Flüssigkeitsspender weist einen Flüssigkeitsspeicher zur Lagerung der Flüssigkeit vor dem Austrag sowie einen gegenüber dem Flüssigkeitsspeicher verlagerbaren Dosierkopf auf. Im Dosierkopf ist eine Dosierkammer zur Aufnahme einer Charge der Flüssigkeit vorgesehen, die zuvor aus dem Flüssigkeitsspeicher in die Dosierkammer gefördert wurde und die dort zum anschließenden Austrag vorgesehen ist. Zur Förderung von Flüssigkeit aus dem Flüssigkeitsspeicher zur Dosierkammer weist der Flüssigkeitsspender eine Pumpeinrichtung auf, welche zwischen dem Flüssigkeitsspeicher und der Dosierkammer angeordnet ist, so dass eine Pumpkammer der Pumpeinrichtung durch Verlagerung des Dosierkopfes in einer Pumprichtung Flüssigkeit in die Dosierkammer fördert.

Erfindungsgemäße Flüssigkeitsspender sind insbesondere vorgesehen für Flüssigkeiten des kosmetischen oder pharmazeutischen Bereichs und können für Flüssigkeiten unterschiedlicher Viskosität Verwendung finden. Besondere Anwendungsfelder, für die die erfindungsgemäßen Flüssigkeitsspender besonders geeignet sind, sind kosmetische und pharmazeutische Cremes. Insbesondere sind erfindungsgemäße Flüssigkeitsspender zur Behandlung der Kopfhaut mit einer Flüssigkeit vorgesehen, insbesondere Stoffe zur Behandlung von Haarausfall (z.B. Minoxidil, 5- Alphareduktasehemmer). Weiterhin ist der erfindungsgemäße Flüssigkeitsspender für lokal oder systemisch wirkende Stoffe (topisch bzw. transdermal) geeignet. Insbesondere handelt es um pharmazeutische Flüssigkeiten zur Behandlung von entzündlichen Erkrankungen der Haut (Dermatitis, Psoriasis, Rosacea) gleich welchen Ursprungs, Infektionen der Haut (Warzen, anderen virale oder bakterielle Infektionen, Parasiten), allergische Reaktionen und Erkrankungen (atopisches Ekzem, allergische Reaktionen der Haut), Erkrankungen, die ganz oder teilweise auf Stoffwechsel- oder Hormonveränderungen zurückzuführen sind (Akne, Hormonersatztherapien, Alopecia, Rosacea), sowie Behandlungen von trockener Haut (Ichthyosis und Vorformen). Hierbei spielen insbesondere die folgenden Molekülgruppen eine Rolle, Ätzmittel (Warzenentfernungsmittel wie Trichloressigsäure, Salicylsäure), Photosensitizer (z.B. Psoralene), Stoffe zur Feuchtigkeitsregulierung (z.B. Harnstoff, Hyaluronsäurederivate), antientzündliche Stoffe (z.B. Zinkderivate, Kortikosteroide), Modulatoren des Immunsystems (z.B. Kortikosteroide, Imiquimod, Calcineurin-Inhibitoren, wie macrolatame, tacrolimus, pimecrolimus), Imidazolin- und Triazol-Derivate (z.B. Brimonidin, Clotrimazol, Ketoconazol, Miconazol), Allylamine-Derivate, antivirale Stoffe (z.B. Docosanol, Purin-Analoga, wie Acyclovir und Pencyclovir), Antiparasitika (z.B. Avermectin-Derivate, Praziquantel), Stoffe zur Versorgung von Wunden (z.B. Salzlösung, HyaluronsäureDerivate, biologische Wirkstoffe), und Wirkstoffkombinationen der traditionellen Medizin.

Der Flüssigkeitsspeicher ist daher im verkaufsfertigen Zustand vorzugsweise mit einer der genannten Flüssigkeiten befüllt.

Die Pumpeinrichtung zur Förderung von Flüssigkeit in den Dosierspeicher wird vom Benutzer vorbereitend für einen nachfolgenden Austrag betätigt. Bei einem erfindungsgemäßen Flüssigkeitsspender ist primär vorgesehen, dass die Befüllung der Dosierkammer zeitlich getrennt vom Vorgang des Austrags stattfindet. Der Benutzer befüllt also die Dosierkammer und beginnt anschließend mit dem Austrag der Flüssigkeit aus der Dosierkammer.

Die Pumpeinrichtung ist vorzugsweise derart ausgebildet, dass der Dosierkopf zum Zwecke des Pumpens gegenüber dem Flüssigkeitsspeicher linear verlagerbar ist, insbesondere entlang einer Pumprichtung, die vorzugsweise einer Mittelachse des üblicherweise länglichen Flüssigkeitsspeichers entspricht. Die Pumpeinrichtung umfasst vorzugsweise in gattungsüblicher Weise eine Pumpkammer, die durch ein Einlassventil und ein Auslassventil phasenweise vom Flüssigkeitsspeicher und der Dosierkammer getrennt ist. Eine hier vorgeschlagene Möglichkeit ist die Gestaltung mit einer flexiblen Pumpkammerwandung, insbesondere in Art eines Balges, die an einer am Flüssigkeitsspeicher vorgesehenen Entnahmeöffnung und an einem Eingang des Dosierkopfes befestigt ist. Eine solche Balgpumpe ist vergleichsweise günstig. Der Pumpenbalg kann je nach Steifigkeit eine separate Rückstellfeder ersetzen. Eine solche kann jedoch dennoch vorgesehen sein.

Der Flüssigkeitsspeicher eines erfindungsgemäßen Flüssigkeitsspenders weist vorzugsweise ein Volumen zwischen 20 ml und 250 ml auf. Die Dosierkammer weist vorzugsweise ein Innenvolumen zwischen 0,1 ml und 10 ml auf, insbesondere vorzugsweise zwischen 0,5 ml und 5 ml. Das Fördervolumen der Pumpeinrichtung je einzelner Betätigung beträgt vorzugsweise zwischen 0,02 ml und 5 ml, wobei das Fördervolumen insbesondere vorzugsweise geringer als das Dosierkammervolumen ist. Ausgehend von einer leeren Dosierkammer bedarf es somit mehrerer Betätigungen der Pumpeinrichtung, um diese vollständig zu füllen. Insbesondere vorzugsweise beträgt das Fördervolumen der Pumpeinrichtung zwischen 20% und 90% des Dosierkammervolumens, insbesondere zwischen 20% und 40%.

Bei einem erfindungsgemäßen Flüssigkeitsspender dient das Fördern von Flüssigkeit in die Dosierkammer insbesondere der Isolation einer Teilcharge aus dem Flüssigkeitsspeicher, ohne dass die Flüssigkeit hierbei einer weitergehenden Änderung unterworfen sein soll. Es ist also insbesondere nicht vorgesehen, dass mehrere Flüssigkeitsspeicher mit unterschiedlichen Flüssigkeiten vorgesehen sind, die mit der Dosierkammer zum Zwecke des dortigen Mischens der Flüssigkeiten verbunden sind. Stattdessen weist ein erfindungsgemäßer Flüssigkeitsspender üblicherweise nur einen Flüssigkeitsspeicher und nur eine Pumpe zwischen diesem und der Dosierkammer auf.

Die Flüssigkeit, die mittels der Pumpeinrichtung in die Dosierkammer gefördert wird, steht dort für die anschließende Aufbringung auf einer Oberfläche, insbesondere der Haut des Benutzers, bereit.

Dabei ist erfindungsgemäß vorgesehen, dass der Austrag der Flüssigkeit aus der Dosierkammer durch Kontakt mit der Oberfläche gesteuert wird, wobei hierfür verschiedene Varianten vorgeschlagen werden.

Gemäß einer ersten Variante der Erfindung verfügt der Flüssigkeitsspender über eine die Dosierkammer begrenzende Applikatorwandung am Dosierkopf, deren Außenseite für den unmittelbaren Körperkontakt vorgesehen ist. Diese Applikatorwandung ist von einer Austragöffnung oder einer Mehrzahl von Austragöffnungen durchdrungen. Die Applikatorwandung ist gegenüber angrenzenden Wandungen der Dosierkammer als Ganzes oder durch Verformung in einer Verlagerungsrichtung verlagerbar. Vorzugsweise weist die Applikatorwandung eine außenseitige Strukturierung zum Einmassieren der ausgetragenen Flüssigkeit auf, beispielsweise in Form einer Mehrzahl von kleinen Erhebungen oder in Form von kreisförmigen Erhebungen.

Durch die Verlagerung und/oder Verformung der Applikatorwandung wird das Volumen der Dosierkammer reduziert, so dass Flüssigkeit durch die mindestens eine Austragöffnung aus der Dosierkammer herausgefördert wird. Diese Verlagerung bzw. Verformung erfolgt durch Andrücken der Außenseite der Applikatorwandung an die Oberfläche, insbesondere die Haut, die mit der Flüssigkeit versehen werden soll.

Die Dosierkammer stellt bei dieser ersten Variante der Erfindung eine Art zweite Pumpkammer dar. Die Austragöffnungen sind vorzugsweise permanent offen, so dass die Flüssigkeit grundsätzlich frei aus der Dosierkammer hier hinausgelangen kann. Allerdings sind sie vorzugsweise derart auf die Flüssigkeit abgestimmt, dass keine oder nur eine geringe Menge Flüssigkeit hier frei herauslaufen würde. Die beschriebene erste Variante der Erfindung ist zudem insbesondere für höherviskose Flüssigkeiten vorgesehen, deren Neigung zum ungewollten Auslaufen recht gering ist. Zudem kann vorgesehen sein, dass die Austragöffnungen durch die Verformung der Applikatorwandung beim Andrücken an die Haut verformt werden und hierdurch gegenüber einem Ruhezustand einen geringeren Fließwiderstand aufweisen. Auch kann vorgesehen sein, dass die mindestens eine Austragöffnung eine Ventilfunktion aufweist, die zumindest ein druckloses Auslaufen von Flüssigkeit aus der Dosierkammer verhindert.

Eine erste mögliche Gestaltung zur Erzielung der Verlagerbarkeit der Applikatorwandung gegenüber den angrenzenden Dosierkammerwandungen des Dosierkopfes sieht vor, dass die Applikatorwandung als Ganzes in der Verlagerungsrichtung verlagerbar ist. Hierfür ist vorzugsweise eine Schiebeführung vorgesehen, bei der eine mit der Applikatorwandung verbundene Führungsfläche vorgesehen ist, die verschiebbar an einer korrespondierenden Führungsfläche des Dosierkopfes anliegt, wobei die Führung vorzugsweise flüssigkeitsdicht ist. Bei einer solchen Gestaltung finden somit zwei im wesentlichen starre Bauteile Verwendung, nämlich eine Applikatorhülse, an der die Applikatorwandung vorgesehen ist, und ein ortsfest zu einer Basis des Dosierkopfes angeordneter Führungsabschnitt. Zwischen dem Führungsabschnitt und der Applikatorhülse ist die Führung vorgesehen. Weiterhin begrenzen die Applikatorhülse und der genannte Führungsabschnitt gemeinsam einen größenveränderlichen Teilbereich der Dosierkammer. Bei einer besonderen Gestaltung dessen kann vorgesehen sein, dass eine Federeinrichtung vorgesehen ist, die die Applikatorwandung bzw. die Applikatorhülse mit Applikatorwandung in Richtung einer äußeren Endlage kraftbeaufschlagt.

Eine zweite mögliche Gestaltung zur Erzielung der Verlagerbarkeit der Applikatorwandung sieht vor, dass die Applikatorwandung als durch elastische Verformung einer Dosierkammerwandung in einer Verlagerungsrichtung auslenkbare Wandung gestaltet ist. Dies kann dadurch erzielt werden, dass die Applikatorwandung, also die zum Kontakt mit der Körperoberfläche bestimmte Wandung, in sich verformbar ist, wobei sie hierfür insbesondere vorzugsweise nach außen gewölbt ist, so dass sie durch Andrücken an eine Oberfläche dosiert verformbar ist. Alternativ oder zusätzlich kann jedoch auch vorgesehen sein, dass die Applikatorwandung fest mit einer anderweitigen Dosierkammerwandung verbunden ist, die ihrerseits elastisch verformbar ist, beispielsweise in Art einer Balgwandung.

Die Verformbarkeit der Applikatorwandung selbst und/oder angrenzender Teile der Dosierkammerwandung gestatten es, das Volumen der Dosierkammer zu verkleinern und hierdurch Flüssigkeit durch die Austragöffnung oder die Austragöffnungen hindurch auszutragen. Durch die Verwendung eines elastischen verformbaren Bauteils, welches die Applikatorwandung oder eine andere verformbare Dosierkammerwandung bildet, ist eine besonders günstige und leicht auslegbare Bauweise möglich, da auf eine Abdichtung an zueinander beweglichen Teilen verzichtet werden kann. So kann im einfachsten Falle eine Balgwandung mit endseitig und einstückig daran angeformter Applikatorwandung mit Austragöffnung vorgesehen sein, die mit dem gegenüberliegenden Ende an einer im übrigen einstückigen oder zweistückigen Dosierkopfbasis befestigt ist. Ergebnis ist eine zuverlässige und günstige Bauweise.

Eine vorteilhafte Ausgestaltung sieht vor, dass die Applikatorwandung als bistabile Applikatorwandung ausgebildet ist, die eine erste und ein zweite stabile Form einnehmen kann, nämlich eine Form mit minimalem Dosierkammervolumen und ein mit maximalem Dosierkammervolumen. Unter der Bistabilität ist zu verstehen, dass die Applikatorwandung bei abweichender Formgebung in eine der beiden stabilen Formen gedrückt wird.

Insbesondere ist die erste Form der Applikatorwandung eine nach außen gewölbte Form, die die Applikatorwandung bei befüllter Dosierkammer einnimmt, und die zweite Formgebung eine nach innen gewölbte Form ist, die die Applikatorwandung bei Volumenreduzierung der Dosierkammer einnimmt. Der Nutzen der Bistabilität liegt insbesondere darin, dass der gegenwärtige Zustand des Flüssigkeitsspenders für den Benutzer erkennbar ist. Ist die Applikatorwandung in ihrer Stellung minimalen Dosierkammervolumens, weiß der Benutzer, dass er vor dem Austrag die Pumpeinrichtung zu betätigen hat. Ist die Applikatorwandung in ihrer Stellung maximalen Dosierkammervolumens, so kann der Austrag unmittelbar beginnen. Durch den Austrag wird die Applikatorwandung dann eingedrückt, so dass der Pumpbedarf vor dem nächsten Austrag wieder ersichtlich ist. Die Bistabilität kann zusätzlich bei ihrem Wechsel in die zweite Formgebung dafür sorgen, dass die Flüssigkeit im an die Applikatorwandung angrenzenden Teil der Dosierkammer vollständig ausgetragen wird. Auch kann bei passender Anordnung der Austragöffnung vorgesehen sein, dass die Austragöffnung bei Anordnung der Applikatorwandung in der zweiten Formgebung verschlossen ist.

Eine bereits oben kurz erwähnte Gestaltung sieht vor, dass die Dosierkammerwandung zumindest abschnittsweise als ein stauchbarer Balgabschnitt ausgebildet ist. Die Applikatorwandung ist an einem Ende des Balgabschnitts vorgesehen, während der Balgabschnitt mit dem anderen Ende an starren Teilen des Dosierkopfes befestigt ist. Durch Andrücken der Applikatorwandung an die Hautoberfläche ist der Balgabschnitt zusammendrückbar und die Dosierkammer hierdurch verkleinerbar, so dass Flüssigkeit durch die mindestens eine Austragöffnung hindurch hinausgedrückt wird.

Die Gestaltung der Dosierkammerwandung in Art eines Balgabschnitts stellt eine günstige Bauweise dar. Weiterhin begünstigt die Balgform eine weitgehend reproduzierbare Verformung, so dass die Applikatorwandung am Ende des Balges in etwa ihre bestimmungsgemäße Ausrichtung beibehält, dabei jedoch in geringem und erwünschtem Maße flexibel ist, so dass sie während der Bewegung des Flüssigkeitsspenders über die Haut an dieser konstant flächig anliegen kann.

Die Bistabilität lässt sich insbesondere dadurch erzielen, dass die Applikatorwandung als solche in eine der beiden stabilen Formen tendiert. Etwas Ähnliches ist jedoch auch zu erzielen, wenn der Dosierkopf eine Rasteinrichtung aufweist, mittels derer die Applikatorwandung nach Entleerung in einer Minimum-Stellung gehalten wird, bei der die Dosierkammer ein minimales Volumen aufweist.

Bei einer solchen Gestaltung ist es nicht die Formgebung der Applikatorwandung als solche, die einen stabilen Zustand der Applikatorwandung im eingedrückten Zustand ermöglicht, sondern die Rasteinrichtung, die durch Rastmittel an der Applikatorwandung oder einem daran vorgesehenen Fortsatz einerseits und einer korrespondierenden Rastkante am Dosierkopf eine entsprechende Fixierung ermöglicht.

Die Rasteinrichtung kann insbesondere derart ausgebildet sein, dass sie durch den durch die Pumpeinrichtung erzeugbaren Druck gelöst werden kann, insbesondere durch anliegenden Flüssigkeitsdruck. Die Rasteinrichtung wird also durch das Befüllen der Dosierkammer mittels der Pumpeinrichtung gelöst, so dass die Applikatorwandung in eine Nutzstellung gelangen kann, insbesondere eine nach außen gewölbte Nutzstellung.

Baulich ist eine Gestaltung der Rasteinrichtung bevorzugt, bei der diese einen Rastkörper aufweist, der mit der Applikatorwandung gemeinsam verlagerbar ist und in einer Endlage mit minimalem Volumen der Dosierkammer mit einer Haltestruktur des Dosierkopfes zusammenwirkt, so dass die Applikatorwandung in der Minimum-Stellung verbleibt.

Insbesondere kann die Rasteinrichtung in einem Flüssigkeitskanal der Dosierkammer vorgesehen sein und diesen verschließen, wenn sich die Applikatorwandung in der Minimum-Stellung befindet. Die Rasteinrichtung bildet in einem solchen Fall auch eine Transportsicherung, die dem Auslaufen des Flüssigkeitsspenders entgegenwirkt. Flüssigkeitsreste, die stromaufwärts der Rasteinrichtung in der Dosierkammer verblieben sind, werden durch die isolierende Rasteinrichtung davon abgehalten, bis zur Austragöffnung zu gelangen. Erst wenn der durch weitere Pumpbetätigungen bewirkte Flüssigkeitsdruck ausreichend hoch ist, löst sich die Rasteinrichtung, insbesondere unter elastischer Verformung des genannten Rastkörpers, so dass die Nutzstellung erzielt wird.

Bei einem Flüssigkeitsspeicher gemäß der beschriebenen ersten Variante ist einerseits der Dosierkopf als Ganzes gegenüber dem Flüssigkeitsspeicher verlagerbar und andererseits die Applikatorwandung zumindest teilweise gegenüber angrenzenden Teilen des Dosierkopfes, insbesondere einer starren Basis des Dosierkopfes. Damit es nicht zu einem ungewollten Pumpvorgang an der Pumpeinrichtung kommt, wenn die Applikatorwandung auf die Hautoberfläche gedrückt wird, können die Verformbarkeit der Applikatorwandung und die für den Pumpvorgang erforderliche Kraft derart aufeinander abgestimmt sein, dass die Verformung der Applikatorwandung und der Flüssigkeitsaustrag von Flüssigkeit aus der Dosierkammer mit geringerem Kraftaufwand als der Pumpvorgang einhergehen.

Bevorzugt ist allerdings eine Gestaltung bei der die Entkoppelung der Verlagerung der Applikatorwandung und des Dosierkopfes anderweitig erfolgt, nämlich indem die Verlagerungsrichtung nicht-parallel zur Pumprichtung ausgerichtet ist. Die bestimmungsgemäße Verlagerungsrichtung der Applikatorwandung, die üblicherweise mit der mittleren Austragrichtung übereinstimmt und/oder in Richtung einer Flächennormalen auf dem Mittelpunkt der Applikatorwandung ausgerichtet ist, ist gegenüber der Pumprichtung angewinkelt, so dass eine Kraftbeaufschlagung der Applikatorwandung in bestimmungsgemäßer Verlagerungsrichtung nicht oder nur in geringerem Maße eine Pumpenbetätigung zur Folge hat. Ein eingeschlossener Winkel zwischen der Verlagerungsrichtung und der Pumprichtung beträgt vorzugsweise mindestens 30°, insbesondere vorzugsweise zwischen 45° und 75°.

Insbesondere kann der eingeschlossene Winkel zwischen der Verlagerungsrichtung und der Pumprichtung derart an eine Führung zwischen dem Dosierkopf und dem Flüssigkeitsspeicher angepasst sein, dass eine Verlagerung des Dosierkopfes gegenüber dem Flüssigkeitsspeicher in Folge einer Kraftbeaufschlagung der Applikatorwandung in Verlagerungsrichtung nicht eintritt. Dies kann insbesondere erzielt werden, indem der Winkel wo gewählt ist, dass im Bereich der Führung mechanische Selbsthemmung eintritt.

Am Dosierkopf ist bei erfindungsgemäßen Flüssigkeitsspendern vorzugsweise eine Betätigungsfläche vorgesehen, insbesondere auf der dem Flüssigkeitsspeicher abgewandten Seite des Dosierkopfes. Durch Kraftbeaufschlagung der Betätigungsfläche in Pumprichtung kann ein Pumpvorgang ausgelöst werden.

Eine besonders bevorzugte Gestaltung sieht dabei vor, dass der Dosierkopf seitlich der Betätigungsfläche einen bezogen auf eine Längsachse die Betätigungsfläche überragenden Applikatordom aufweist, an dem die Applikatorwandung vorgesehen ist. Der Applikatordom ist ein Abschnitt, der sich seitlich der Betätigungsfläche über diese erhebt und an dem, insbesondere an dessen der Betätigungsfläche abgewandter Seite, die verlagerbare Applikatorwandung vorgesehen ist.

Die seitlich des Applikatordoms angeordnete Betätigungsfläche ermöglicht durch ihre Anordnung einen insgesamt kompakteren Aufbau des Flüssigkeitsspenders. Zudem ist die Pumpeinrichtung des Flüssigkeitsspenders aufgrund der nah am Flüssigkeitsspeicher angeordneten Betätigungsfläche gut zu betätigen.

Von besonderem Vorteil kann es sein, wenn der Applikatordom sich bezogen auf eine Querrichtung bis über die Betätigungsfläche erstreckt. Die Betätigungsfläche ist bei einer solchen Gestaltung gleichsam in einer Art Tasche des Dosierkopfes vorgesehen. Oberhalb der Betätigungsfläche erstreckt sich der Applikatordom von der Seite aus über diese hinüber. Auch hierdurch wird ein kompakter Aufbau des Flüssigkeitsspenders begünstigt, der eine vergleichsweise große Applikatorwandung mit vergleichsweise geringen Außenmaßen des Flüssigkeitsspenders kombiniert.

Gemäß einer zweiten Variante der Erfindung verfügt der Dosierkopf über eine die Dosierkammer begrenzende Dosierkammerwandung, die gegen eine Federkraft auslenkbar ist. Der Dosierkopf verfügt weiterhin über mindestens eine Austragöffnung und ein der Austragöffnung zugeordnetes Schaltventil, welches für den unmittelbaren Oberflächen- und insbesondere Hautkontakt vorgesehen ist und welches durch Kraftbeaufschlagung geöffnet werden kann, so dass Flüssigkeit aus der Dosierkammer unter Entspannung der auslenkbaren Dosierkammerwandung durch die Austragöffnung hindurch ausströmen kann.

Anders als bei der ersten Variante der Erfindung gleicht die Wirkung des Andrückens des Dosierkopfes an die Haut nicht einerArt weiterem Pumpvorgang, sondern dem Öffnen eines Ventils. Durch die Betätigung der Pumpvorrichtung wird bei dieser zweiten Gestaltung eine Dosierkammerwandung elastisch ausgelenkt, nämlich gegen die Kraft eines separaten elastischen Federmittels oder gegen die Kraft der sich spannenden Dosierkammerwandung. Die Flüssigkeit in der Dosierkammer steht somit unter Druck.

Wird nun der Dosierkopf gegen die Haut gedrückt, so wird hierdurch das Schaltventil geöffnet und die druckbeaufschlagte Flüssigkeit aus der Dosierkammer strömt durch die Austragöffnung aus.

Die grundsätzliche Bauweise in Hinblick auf die Betätigungsfläche und den Applikatordom unterscheidet sich nicht zwischen der ersten und der zweiten Variante der Erfindung. Die oben zur ersten Variante genannten bevorzugten baulichen Aspekte gelten daher auch für die zweite Variante der Erfindung.

Insbesondere vorzugsweise ist die auslenkbare Dosierkammerwandung unmittelbar im Bereich der Austragöffnung vorgesehen. Sie weist hierfür vorzugsweise eine Durchbrechung auf, durch die hindurch die Flüssigkeit zur Austragöffnung strömen kann. Die Durchbrechung kann auch selbst die Austragöffnung darstellen.

Das Schaltventil weist vorzugsweise einen Ventilkörper auf, der verschlieblich in der Austragöffnung angeordnet ist und der in einer äußeren Endlage, in die er durch Flüssigkeitsdruck in der Dosierkammer gedrückt wird, die Austragöffnung verschließt. Dieser Ventilkörper ist von außen eindrückbar, insbesondere indem er selbst gegen die Hautoberfläche gedrückt wird, hierdurch tiefer in die Austragöffnung geschoben wird und dabei die Austragöffnung partiell freigibt, so dass der Flüssigkeitsaustrag ermöglicht wird.

Es wird als vorteilhaft angesehen, wenn die elastisch auslenkbare Wandung von außen nicht zugänglich ist. Es ist daher vorzugsweise innerhalb des Dosierkopfes ein Raum vorgesehen, in den die Dosierkammerwandung unter dem Eindruck des Flüssigkeitsdrucks in der Dosierkammer ausgelenkt werden kann. Insbesondere handelt es sich um einen unterhalb einer Applikatorwandung liegenden Ringraum, der hierfür vorgesehen ist.

Gemäß einer dritten Variante der Erfindung verfügt der Flüssigkeitsspender über mindestens eine sich an die Dosierkammer anschließende Austragöffnung am Dosierkopf zum Austrag von Flüssigkeit. Diese Austragöffnung weist bestimmungsgemäß beim Austrag nach unten, so dass die Flüssigkeit aus der Dosierkammer grundsätzlich aus dieser herausfließen kann. Diese Austragöffnung ist dabei jedoch derart gestaltet und mit der Dosierkammer verbunden, dass aus der Dosierkammer nach unten in Richtung der Austragöffnung strömende Flüssigkeit durch die Oberflächenspannung der Flüssigkeit an der Austragöffnung verbleibt, bis die Oberflächenspannung durch Kontakt mit einer Körperoberfläche unterbrochen wird.

Die geometrische Gestaltung der Austragöffnung ist also an die Gewichtskraft der Flüssigkeit bei befüllter Dosierkammer und die Eigenschaften der Flüssigkeit derart angepasst, dass die Flüssigkeit nicht frei herausfließen kann, jedoch soweit an die Austragöffnung herankommt, dass die sich dort bildende Flüssigkeitsoberfläche von außen gestört werden kann. Zur Verhinderung des Herausfließens und zur Bildung der Flüssigkeitsoberfläche unmittelbar an der Austragöffnung sind verschiedene Maßnahmen möglich, die einzeln oder in Kombination Verwendung finden, so insbesondere die Gestaltung der Austragöffnung als kreisrunde Austragöffnung, ein lichter Querschnitt an der Austragöffnung von maximal 3 mm, insbesondere maximal 2 mm sowie einen die Austragöffnung umgebenden ringförmigen Anhaftbereich, dessen Breite vorzugsweise mindestens 1 mm beträgt.

Um zu gewährleisten, dass die Flüssigkeit nicht frei aus der Austragöffnung bzw. den Austragöffnungen ausströmt, ist vorzugsweise weiterhin vorgesehen, dass der Dosierkopf mindestens eine schlanke Applikatorspitze aufweist, an deren distalem Ende die mindestens eine Austragöffnung vorgesehen ist. Der enge Kanal innerhalb dieser Spitze verringert ebenfalls die Neigung der Flüssigkeit, frei aus der Austragöffnung herauszufließen. Die Länge der mindestens einen Applikatorspitze beträgt vorzugsweise zwischen 5 mm und 20 mm, insbesondere vorzugsweise mindestens 10 mm und/oder höchstens 15 mm. Der Innendurchmesser der Applikatorspitze beträgt vorzugsweise zwischen 1 mm und 5 mm, wobei der Innendurchmesser sich vorzugweise von der Austragöffnung bis zu einem Hauptbereich der Dosierkammer aufweitet, insbesondere vorzugsweise mit einem Öffnungswinkel zwischen 1° und 15°.

Die Anpassung der Austragöffnung und ggf. der Applikatorspitze an die Flüssigkeit erfolgt vorzugsweise derart, dass die Flüssigkeit mit ihrer Flüssigkeitsoberfläche bis zur Austragöffnung gelangt, hier aber nicht mehr frei herausfließen kann. So ist es möglich, durch Kontakt der hier ausgebildeten Flüssigkeitsoberfläche mit einer Hautoberfläche die Flüssigkeitsoberfläche zu stören und dann das freie Ausfließen bei gleichzeitiger Bewegung des Flüssigkeitsspenders über die Haut zu ermöglichen.

Vorzugsweise ist bei einem solchen Flüssigkeitsspender gemäß der dritten Variante der Erfindung nicht nur eine Austragöffnung vorgesehen, sondern eine Mehrzahl von Austragöffnungen. In diesem Falle sind die Austragöffnungen und deren Zulauf von der Dosierkammer, insbesondere also die Geometrie der Applikatorspitze, vorzugweise derart gestaltet, dass auch dann, wenn durch eine erste der Austragöffnungen Luft frei in die Dosierkammer strömen kann, aus der Dosierkammer nach unten in Richtung einer zweiten der Austragöffnungen strömende Flüssigkeit durch die Oberflächenspannung der Flüssigkeit an der Austragöffnung verbleibt, bis die Oberflächenspannung durch Kontakt mit einer Körperoberfläche unterbrochen wird.

Im Falle mehrerer Austragöffnungen weist der Dosierkopf eine Mehrzahl von separaten Applikatorspitzen auf, die parallel zueinander ausgerichtet sind und an deren Ende jeweils eine Austragöffnung vorgesehen ist. Diese mehreren Applikatorspitzen sind vorzugsweise kammartig nebeneinander angeordnet und erstrecken sich im Wesentlichen parallel zueinander.

Bei einer möglichen Gestaltung ist vorgesehen, dass die mindestens eine Applikatorspitze eine Haupterstreckungsrichtung aufweist, die mit der Pumprichtung übereinstimmt. Anders als bei der ersten und der zweiten Variante der Erfindung ist bei dieser dritten Variante für den Austrag nicht erforderlich, dass der Dosierkopf mit relevanter Kraft an die Oberfläche angedrückt wird. Eine versehentliche Pumpbetätigung ist daher nicht wahrscheinlich.

Dennoch kann es alternativ auch vorgesehen sein, dass die mindestens eine Applikatorspitze eine Haupterstreckungsrichtung und/oder zumindest eine Erstreckungsrichtung im Bereich der Austragöffnung aufweist, die gegenüber der Pumprichtung angewinkelt ist, vorzugsweise um einen Winkel von mindestens 30°, insbesondere vorzugsweise um mehr als 60°.

Auch bei dieser dritten Variante eines Flüssigkeitsspeichers ist vorzugsweise vorgesehen, dass am Dosierkopf eine Betätigungsfläche vorgesehen ist. Eine besonders bevorzugte Variante sieht vor, dass der Dosierkopf an einer dem Flüssigkeitsspeicher abgewandten Seite eine Betätigungsfläche zum Niederdrücken des Dosierkopfes aufweist, wobei die mindestens eine Austragöffnung, insbesondere die mindestens eine Applikatorspitze oder die Gesamtheit der Applikatorspitzen, neben der Betätigungsfläche und exzentrisch zu einer Mittelachse des Dosierkopfes vorgesehen ist. Die Betätigung der Pumpeinrichtung erfolgt bei dieser Gestaltung vorzugsweise mit nur einem auf der Betätigungsfläche aufliegenden Finger.

Eine alternative Gestaltung sieht vor, dass der Dosierkopf an einer dem Flüssigkeitsspeicher abgewandten Seite eine Betätigungsfläche mit zwei Betätigungsteilflächen aufweist, wobei mindestens eine Austragöffnung, insbesondere mindestens eine Applikatorspitze mit daran vorgesehener Austragöffnung, zwischen den beiden Betätigungsteilflächen vorgesehen ist. Bei einer solchen Gestaltung ist vorgesehen, dass zwei Finger des Benutzers bei Betätigung der Pumpeinrichtung auf den beiden Betätigungsteilflächen aufliegen.

### KURZBESCHREIBUNG DER ZEICHNUNGEN

Weitere Vorteile und Aspekte der Erfindung ergeben sich aus den Ansprüchen und aus der nachfolgenden Beschreibung von bevorzugten Ausführungsbeispielen der Erfindung, die nachfolgend anhand der Figuren erläutert sind.
Fig. 1 bis 9 zeigen einen Flüssigkeitsspender gemäß der ersten Variante der Erfindung.
Fig. 1 und 2 zeigen einen Flüssigkeitsspender gemäß der ersten Variante der Erfindung und dessen Dosierkopf in einer ersten Ansicht von außen.
Fig. 3A bis 3D sowie Fig. 4 verdeutlichen die grundsätzliche Funktionsweise von Flüssigkeitsspendern gemäß der ersten Variante der Erfindung.
Fig. 5A bis 5C zeigen den Flüssigkeitsspender der Fig. 3A bis 3D mit vergrößert dargestelltem Dosierkopf.
Fig. 6A bis 6C zeigen eine erste alternative Gestaltung des Flüssigkeitsspenders gemäß der ersten Variante der Erfindung.
Fig. 7A bis 7C zeigen eine zweite alternative Gestaltung des Flüssigkeitsspenders gemäß der ersten Variante der Erfindung.
Fig. 8A bis 8C zeigen eine dritte alternative Gestaltung des Flüssigkeitsspenders gemäß der ersten Variante der Erfindung.
Fig. 9A bis 9D zeigen eine vierte alternative Gestaltung des Flüssigkeitsspenders gemäß der ersten Variante der Erfindung.
Fig. 10A bis 10C zeigen einen Flüssigkeitsspender gemäß der zweiten Variante der Erfindung.
Fig. 11 bis 16 zeigen Flüssigkeitsspender gemäß der dritten Variante der Erfindung.

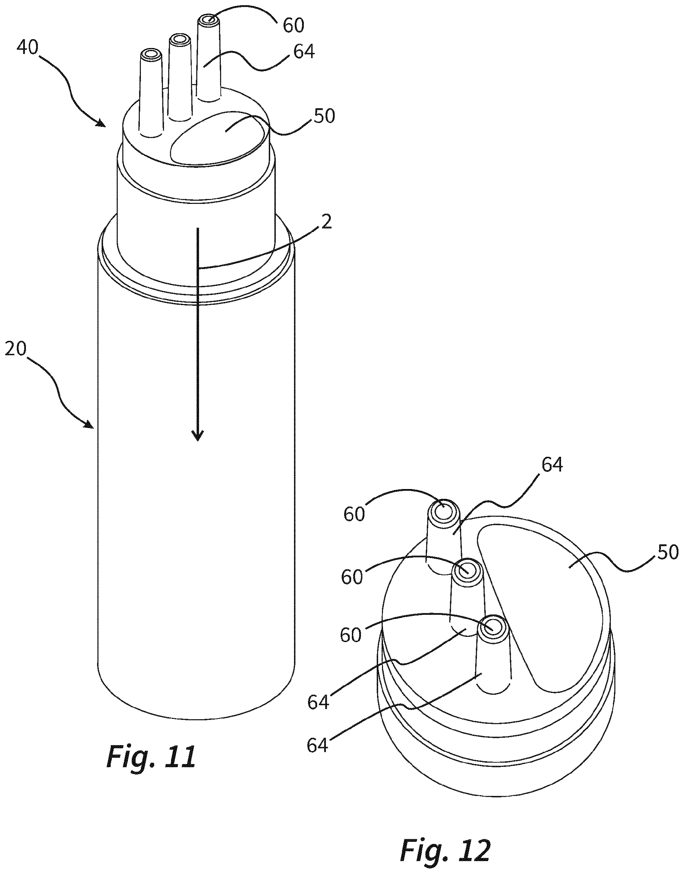

Fig. 11 und 12 zeigen einen Flüssigkeitsspender gemäß der dritten Variante der Erfindung und dessen Dosierkopf in einer ersten Ansicht von außen.

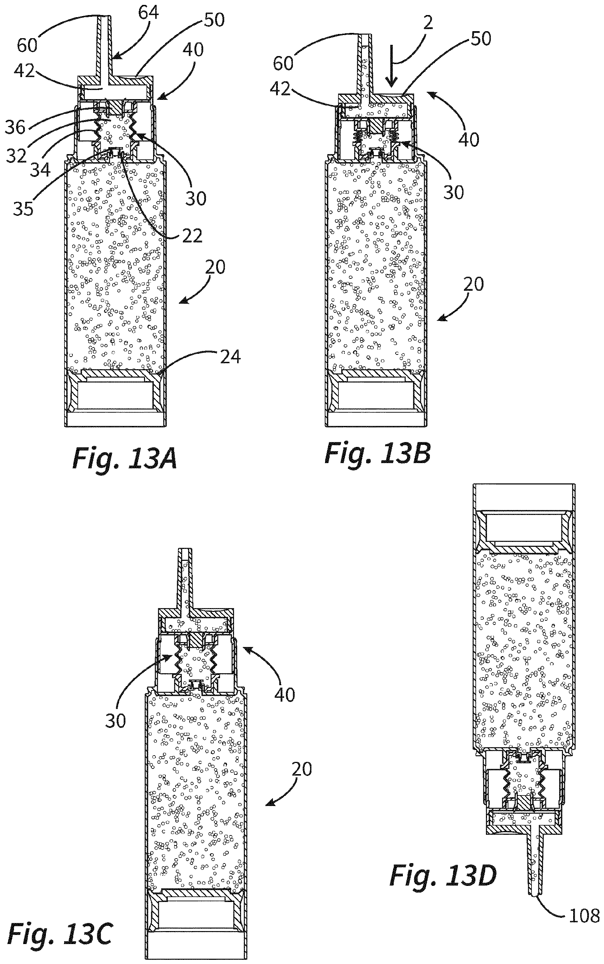

Fig. 13A bis 13D sowie Fig. 14 und Fig. 15A bis 15C verdeutlichen die grundsätzliche Funktionsweise von Flüssigkeitsspendern gemäß der dritten Variante der Erfindung.

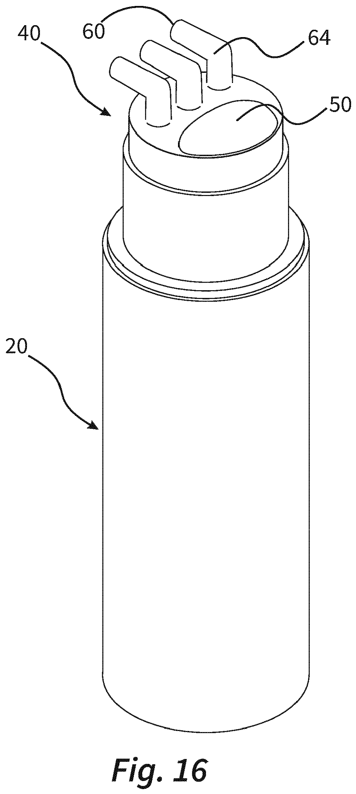

Fig. 16 zeigt eine alternative Gestaltung des Flüssigkeitsspenders gemäß der dritten Variante der Erfindung.

### DETAILLIERTE BESCHREIBUNG DER AUSFÜHRUNGSBEISPIELE

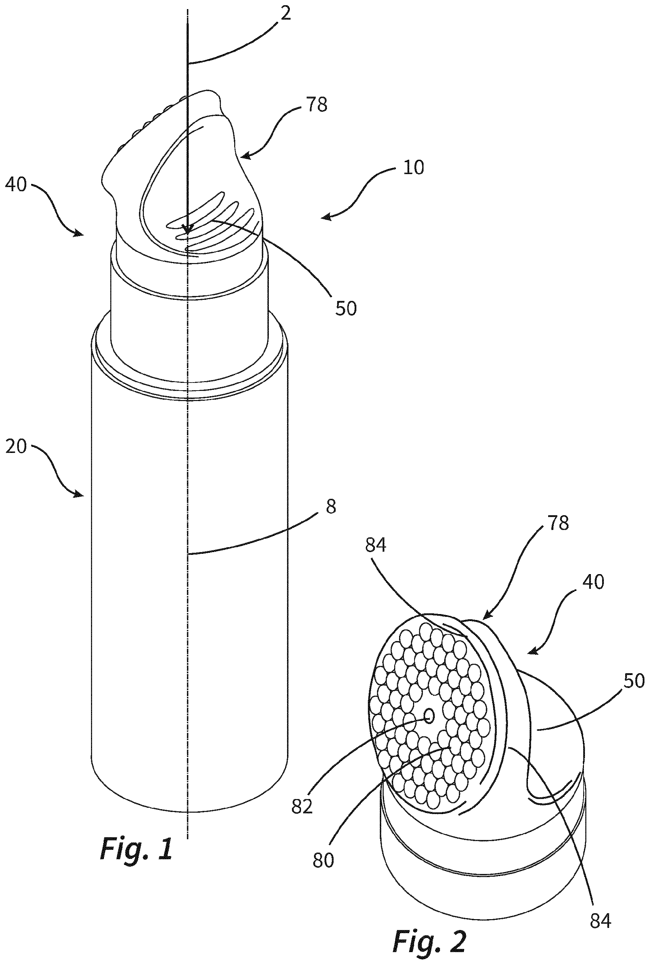

Die Fig. 1 und 2 zeigen einen erfindungsgemäßen Flüssigkeitsspender 10 in einer ersten Variante der Erfindung. Der Flüssigkeitsspender 10 verfügt über einen Flüssigkeitsspeicher 20, an dessen oberem Ende ein Dosierkopf 40 vorgesehen ist. Dieser Dosierkopf 40 ist gegenüber dem Flüssigkeitsspeicher 20 in einer Pumprichtung 2 beweglich, die mit einer Haupterstreckungsrichtung oder Mittelachse 8 des Flüssigkeitsspeichers 20 übereinstimmt. Zum Niederdrücken des Dosierkopfs 40 gegenüber dem Flüssigkeitsspeicher 20 ist eine in Fig. 1 ersichtliche Betätigungsfläche 50 vorgesehen. Neben der Betätigungsfläche 50 befindet sich ein insbesondere auch der Fig. 2 zu entnehmender Applikatordom 78. Dieser weist auf der von der Betätigungsfläche 50 wegweisenden Seite eine mit vielen Erhebungen zum Zwecke der Massage versehene Applikatorwandung 80 auf, die von mindestens einer Austragöffnung 82 durchdrungen ist. Die Applikatorwandung 80 ist in im Weiteren noch erläuterter Weise gegenüber umgebenden Dosierkammerwandungen 84 eindrückbar.

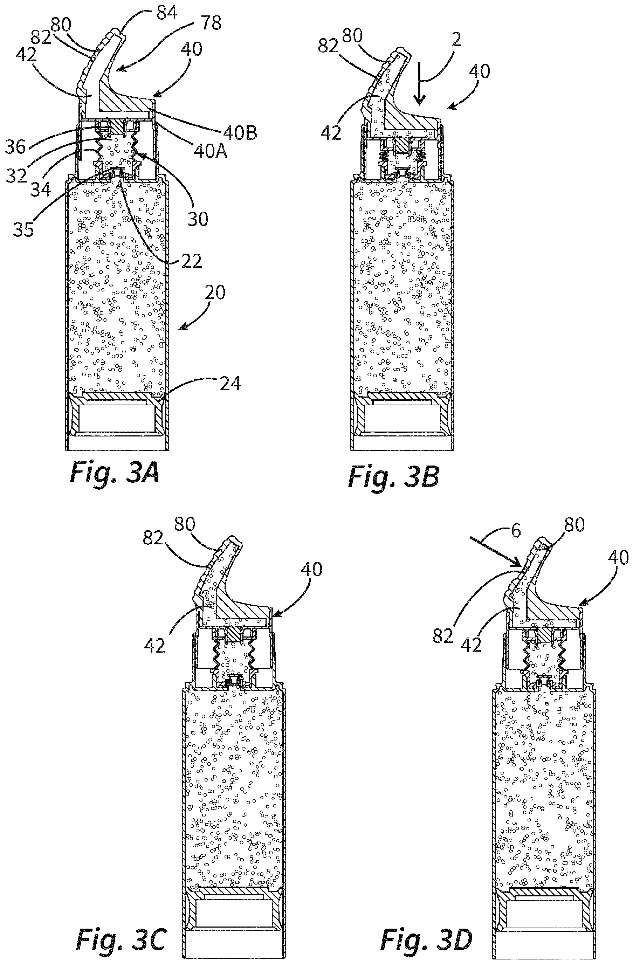

Die Fig. 3A bis 3D verdeutlichen in Schnittdarstellung den Aufbau des Flüssigkeitsspenders 10 der Fig. 1 und 2 und dessen grundsätzliche Funktionsweise im Detail.

Fig. 3A zeigt einen Ausgangszustand. Zu erkennen ist, dass der Flüssigkeitsspeicher 20 zum überwiegenden Teil aus einer zylindrischen Wandung besteht, in die vom unteren Ende aus ein Schleppkolben 24 eingesetzt ist. Am oberen Ende des Flüssigkeitsspeichers 20 ist eine Entnahmeöffnung 22 vorgesehen. Oberhalb dieser Entnahmeöffnung 22 befindet sich eine Pumpeinrichtung30, die vorliegend insbesondere aus einem Pumpkammerbauteil besteht, welches neben einer balgförmigen Pumpkammerwandung 34 auch die beiden Ventilkörper des Einlassventils 35 und des Auslassventils 36 einstückig umfassen kann. Alternativ können die Ventilkörper auch durch separate Ventilkörper gebildet sein. Durch das Einlassventil 35 und das Auslassventil 36 ist eine Pumpkammer 32 begrenzt.

Der Fig. 3A weiterhin zu entnehmen ist, dass der Dosierkopf 40 aus zwei Bauteilen 40A, 40B zusammengesetzt ist, die gemeinsam eine Dosierkammer 42 begrenzen. Diese Dosierkammer 42 erstreckt sich vom Auslassventil 36 bis hinter die Applikatorwandung 80. Das unter Bauteil 40A der beiden Bauteile stellt eine Basis 40A dar, die mit dem den Flüssigkeitsspeicher bildenden Bauteil eine Schiebeführung 38 bildet. Das andere Bauteil 40B ist aus einem elastischeren Kunststoff hergestellt und bildet unter anderem die Applikatorwandung 80.

Im Zustand der Fig. 3A ist die Dosierkammer 42 leer. Um den Flüssigkeitsspender zu verwenden und mit ihm Flüssigkeit auszutragen, wird zunächst Flüssigkeit in die Dosierkammer 42 gepumpt. Dies passiert in der durch Fig. 3B verdeutlichten Weise, indem die Betätigungsfläche 50 und damit der Dosierkopf 40 in Pumprichtung 2 niedergedrückt wird. Hierbei schließt das Einlassventil 35 und das Auslassventil 36 der Pumpeinrichtung 30 öffnet, so dass Flüssigkeit in die Dosierkammer 42 gelangt. Die Volumina der Pumpkammer 32 und der Dosierkammer 42 sind vorliegend derart abgestimmt, dass etwa dreimal die Pumpeinrichtung 30 betätigt werden muss, um die Dosierkammer 42 vollständig zu füllen. In der bei Pumpen üblichen Art und Weise und in der durch Fig. 3C verdeutlichten Weise füllt sich bei geschlossenem Auslassventil 36 und geöffnetem Einlassventil 35 die Pumpkammer 32, wenn der Dosierkopf 40 in entgegengesetzte Richtung unter dem Eindruck der Federkraft der Pumpkammerwandung 34 zurück in seine Ausgangslage gelangt.

Der Zustand der Fig. 3C ist der Zustand, von dem ausgehend ein Austrag der Flüssigkeit möglich ist. Hierzu wird in der durch die Fig. 3D verdeutlichten Weise eine Kraft auf die Applikatorwandung 80 bewirkt, die die Dosierkammer 42 verkleinert, so dass Flüssigkeit durch die Austragöffnung 82 nach außen gelangt.

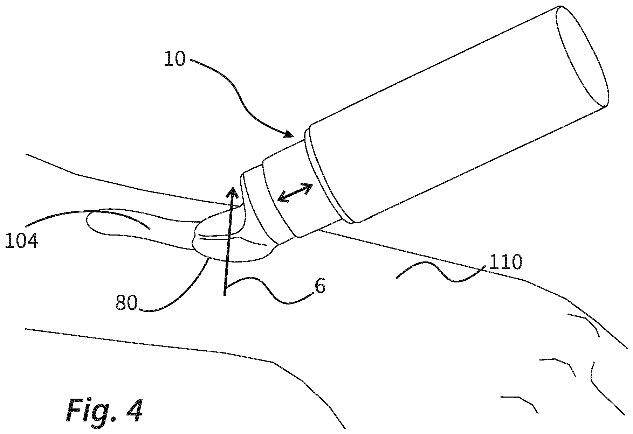

Fig. 4 zeigt, wie dies in der Praxis geschieht. Nachdem der Zustand der Fig. 3C erreicht wird, nimmt der Benutzer den Flüssigkeitsspender in die Hand und führt ihn über eine Hautoberfläche, während er mit sanft ansteigendem Druck die Applikatorwandung 80 eindrückt und hierdurch einen Flüssigkeitsfilm 104 auf der Haut abgibt. Ist die Applikatorwandung 80 vollständig eingedrückt, so verringert der Benutzer kurz den Druck auf die Hautoberfläche, so dass die Applikatorwandung 80 wieder ihre nach außen gewölbte Stellung einnimmt. Hierdurch strömt innerhalb der Dosierkammer 42 Flüssigkeit nach, so dass der Austragvorgang anschließend durch erneutes Eindrücken der Applikatorwandung 80 fortgesetzt werden kann.

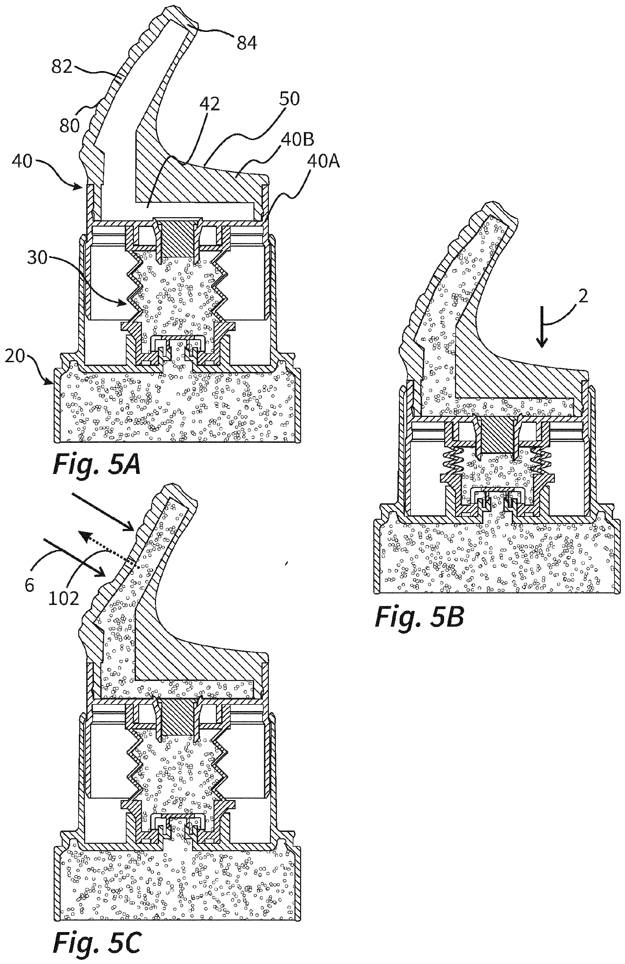

Die Fig. 5A bis 5C zeigen nochmals in vergrößerter Darstellung den Dosierkopf 40 entsprechend den Fig. 3A bis 3D. Hierbei entspricht der Zustand der Fig. 5A jenem der Fig. 3A, der Zustand der Fig. 5B jenem der Fig. 3B und der Zustand der Fig. 5C jenem der Fig. 3D.

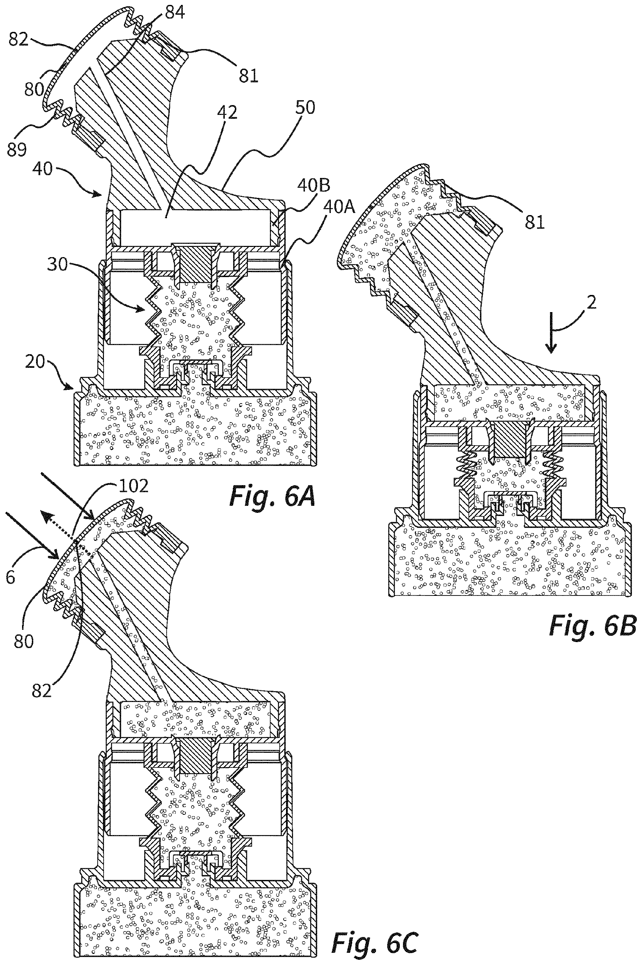

Die Fig. 6A bis 6C zeigen eine alternative Gestaltung. Hier besteht der Dosierkopf 40 nicht nur aus den bereits genannten Bauteilen 40A, 40B, wobei in diesem Falle beide Bauteile 40A, 40B aus starrem Kunststoff bestehen, sondern umfasst weiterhin ein Balgbauteil 81, welches neben einem Balgabschnitt 89 die Applikatorwandung 80 und die Austragöffnung 82 aufweist. Fig. 6A zeigt einen Zustand mit noch nicht befüllter Dosierkammer 42. Der Balg befindet sich in einem komprimierten Zustand, welches bei Abwesenheit einer externen Kraftbeaufschlagung der Grundzustand des Balgabschnitts 89 ist. Wird nun die Pumpeinrichtung 30 durch Niederdrücken des Dosierkopfs 40 betätigt, so strömt innerhalb von mehreren Pumpvorgängen Flüssigkeit aus dem Flüssigkeitsspeicher 20 in die Dosierkammer 42, wobei der Balgabschnitt 89 sich längt und hierdurch einen unmittelbar unter der Applikatorwandung 80 angeordneten Teilraum der Dosierkammer vergrößert. Die Flüssigkeit strömt in dieser Phase nicht oder nur in vernachlässigbarem Umfang bereits durch die Austragöffnung 82 hindurch, da diese einen Öffnungsdruck verlangt, der erst nach vollständiger Längung des Balgabschnitts 89 auftreten kann. Durch die Längung des Balgabschnitts 89 erkennt der Benutzer im Zustand der Fig. 6B, dass die Dosierkammer vollständig befüllt ist. In der durch Fig. 6C verdeutlichten Weise kann er dann durch Andrücken der Applikatorwandung 80 an die Haut die Flüssigkeit aus der Dosierkammer 42 durch die Austragöffnung 82 hindurch ausgeben.

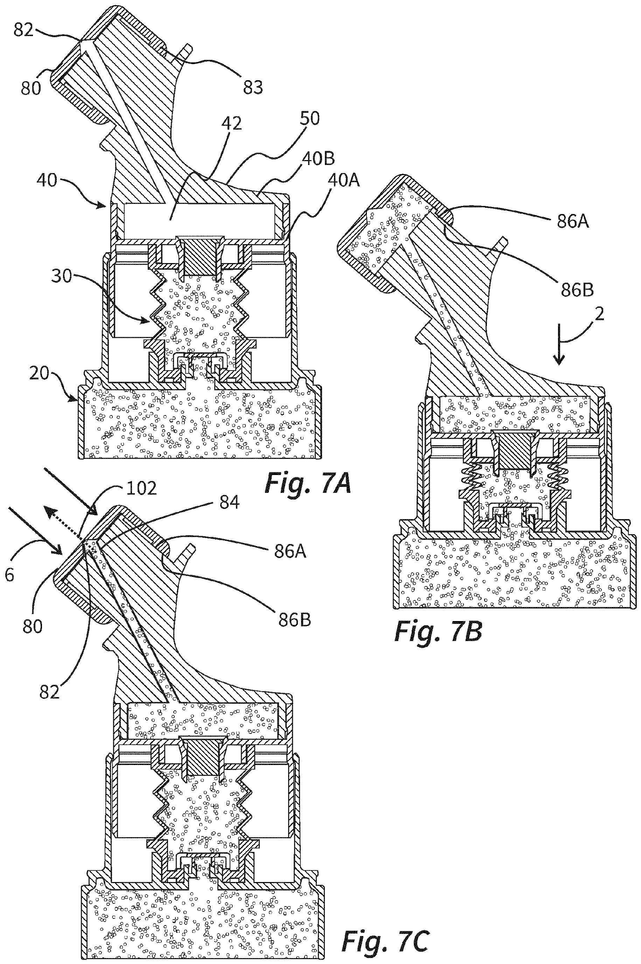

Die Gestaltung der Fig. 7A bis 7C ist ähnlich jener der Fig. 6A bis 6C aufgebaut. Allerdings ist hier kein Balgabschnitt vorgesehen, sondern stattdessen eine starre Applikatorhülse 83, die die Applikatorwandung 80 und die Austragöffnung 82 umfasst und die durch Führungsflächen 86A, 86B verschieblich an einem Führungsabschnitt des Bauteils 40B des Dosierkopfs 40 angebracht ist. Wie die Fig. 7B verdeutlicht, kann durch Betätigen der Pumpeinrichtung 30 ein Bereich unterhalb der Applikatorwandung 80 vergrößert werden, indem die Applikatorhülse 83 schrittweise im Zuge der Pumpbetätigung nach außen geschoben wird. Ist somit die Dosierkammer maximal vergrößert, wie im Zustand der Fig. 7B zu erkennen, so kann anschließend in der bereits beschriebenen Art und Weise die Applikatorwandung 80 gegen eine Hautoberfläche gedrückt werden, um die darin befindliche Flüssigkeit dosiert auszugeben, wie die Pfeile 102 verdeutlichen.

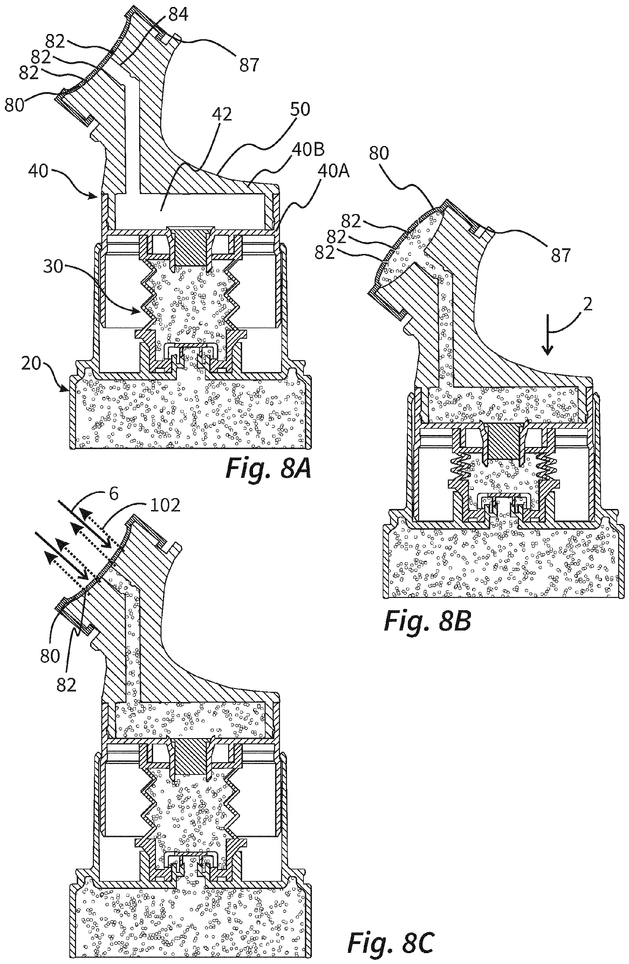

Bei der Gestaltung gemäß der Fig. 8A bis 8C ist vorgesehen, dass am Dosierkopf 40, dessen Bauteil 40B an einem distalen Ende eine schalenförmige Formgebung aufweist, eine elastisch verformbare Applikatorhülse 87 aufgezogen ist, welche auch die Applikatorwandung 80 und die in diesem Fall mehreren Austragöffnungen 82 umfasst. Die Applikatorwandung 80 weist bei dieser Gestaltung eine gewölbte Formgebung auf, die die Neigung hat, in eine von zwei Stellungen zu springen, nämlich einerseits die Minimalstellung der Fig. 8B bei entleerter Dosierkammer 42 und andererseits die Maximalstellung der Fig. 8B bei vollständig befüllter Dosierkammer 42. Im Zustand der Fig. 8A sind die Austragöffnungen 82 durch unmittelbare Anlage am Bauteil 40B verschlossen. Wenn nun ein Pumpvorgang begonnen wird, so ist die Dosierkammer42 nach mehreren Pumphüben ausreichend befüllt, so dass der Flüssigkeitsdruck die Applikatorwandung 80 nach außen drückt und diese die Stellung der Fig. 8B einnimmt. Zwar ist denkbar, dass geringe Flüssigkeitsmengen hierbei bereits ausgetragen werden, dies stört in der Praxis jedoch nicht.

Der überwiegende Teil der Flüssigkeit wird erst ausgetragen, wenn in der durch Fig. 8C verdeutlichten Weise eine Anpresskraft in Verlagerungsrichtung 6 auf die Applikatorwandung 80 wirkt und diese dadurch wieder zurück in die Ausgangsstellung umschlagen lässt. Hierbei wird die unterhalb der Applikatorwandung 80 angeordnete Flüssigkeit in der Dosierkammer 42 durch die Austragöffnungen 82 ausgedrückt, wie die Pfeile 102 verdeutlichen. Der hierdurch erzielte teilentleerte Zustand, der in Fig. 8C dargestellt ist, zeigt dem Benutzer, dass er für einen folgenden Austragvorgang zunächst nochmals die Pumpeinrichtung 30 zu betätigen hätte.

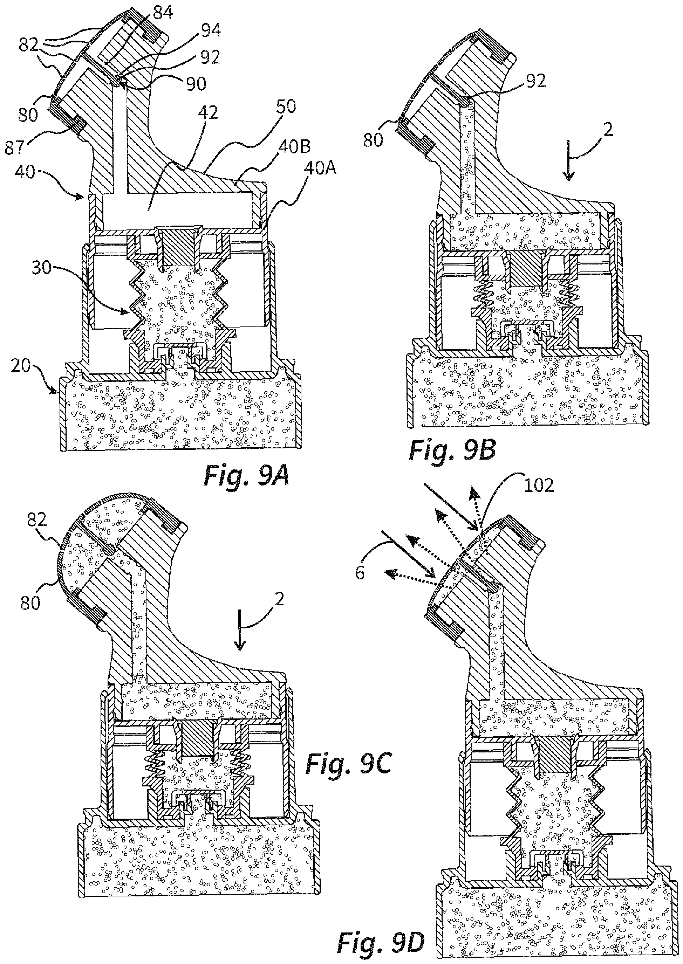

Die Gestaltung der Fig. 9A bis 9C zeigt wiederum einen Dosierkopf 40, auf den ein elastischer Applikatorhülse 87 aufgezogen ist. Diese weist an einem nach innen weisenden Fortsatz einen Rastkörper 92 auf, der in der Ausgangsstellung der Fig. 8B durch eine Haltestruktur 94 am Bauteil 40B gehalten wird, so dass sich die Applikatorwandung 80 in einem flachgezogenen Zustand befindet. Erfolgt nun eine Pumpbetätigung, so wird Flüssigkeit in die Dosierkammer 42 gefördert. Die Fig. 9B zeigt einen Zustand mit teilweise gefüllter Dosierkammer 42. Wird der Flüssigkeitsspender mit einer derart halb befüllten Dosierkammer 42 gehandhabt, so besteht zunächst keine Gefahr, dass Flüssigkeit auslaufen kann, da die Flüssigkeit nicht oder nicht in relevanten Mengen am Rastkörper 29 vorbeiströmen kann.

Erst wenn ausgehend vom Zustand der Fig. 9B nochmals eine Pumpbetätigung stattfindet, so drückt der Flüssigkeitsdruck der bereits in der Dosierkammer 42 befindlichen Flüssigkeit den Rastkörper 92 aus der Haltestruktur 94 heraus, so dass die Applikatorwandung 80 entsprechend der Fig. 9B eine deutlich weiter gewölbte Formgebung einnimmt. Dies ist die Nutzungsstellung, von der ausgehend der Nutzer Flüssigkeit austrägt, indem er in der durch Fig. 9D verdeutlichen Weise die Applikatorwandung 80 in Verlagerungsrichtung 6 durch Andrücken an die Haut kraftbeaufschlagt. Hierdurch wird die Flüssigkeit aus dem unter der Applikatorwandung 80 angeordneten Teil der Dosierkammer hinausgedrückt, wie die Pfeile 102 verdeutlichen.

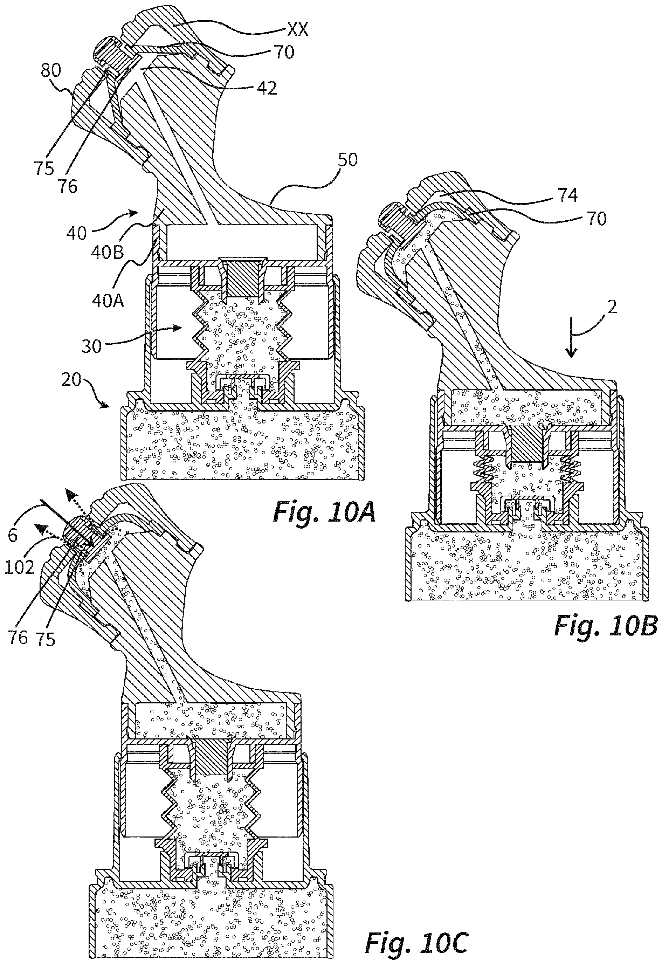

Die Gestaltung der Fig. 10A bis 10C unterscheidet sich von den vorangegangen Ausführungsformen dadurch, dass hier vorgesehen ist, dass die Flüssigkeit in der Dosierkammer 42 druckbeaufschlagt wird. Hierzu weist der Austragkopf eine elastisch auslenkbare Dosierkammerwandung 70 auf, die zentrisch durch eine Öffnung unterbrochen ist, in der sich ein Schaltventilkörper 76 befindet. Wird bei dieser Gestaltung die Pumpeinrichtung 30 betätigt, so gelangt Flüssigkeit in die Dosierkammer 42. Wenn nach vollständiger Befüllung der in Fig. 10A dargestellten Bereiche der Dosierkammer weitergepumpt wird, so drückt der Flüssigkeitsdruck den Schaltventilkörper 76 in eine Schließstellung, so dass keine Flüssigkeit aus der Austragöffnung hindurchtreten kann. Das zusätzlich in die Dosierkammer geförderte Volumen sorgt dann dafür, dass die Dosierkammerwandung elastisch ausgelenkt wird und die Flüssigkeit in der Dosierkammer druckbeaufschlagt. Es ist ein ringförmiger Aufnahmeraum 74 vorgesehen, um die Dosierkammerwandung 70 im ausgelenkten Zustand aufzunehmen. Ausgehend vom Zustand der Fig. 10B wird dann die Nutzung des Flüssigkeitsspenders dadurch eingeleitet, dass der Schaltventilkörper 76 gegen eine Hautoberfläche gedrückt wird und hierdurch in Verlagerungsrichtung 6 eingedrückt wird. Hierdurch verliert er seinen Berührkontakt mit der elastischen Dosierkammerwandung 70 und öffnet dadurch, so dass die Flüssigkeit durch die Austragöffnung 72 hindurch ausströmen kann, wie die Pfeile 102 verdeutlichen.

Die Fig. 11 und 12 zeigen einen Flüssigkeitsspender 10 gemäß der dritten Variante der Erfindung. Hier ist der Dosierkopf 40 mit drei Austragöffnungen 60 versehen, die jeweils am Ende von zueinander parallel ausgerichteten Applikatorspitzen 64 angeordnet sind. Die Applikatorspitzen sind exzentrisch am Dosierkopf 40 vorgesehen, so dass neben ihnen noch eine Betätigungsfläche 50 verbleibt.

Die Fig. 13A bis 13D verdeutlichen auch hier die grundsätzliche Funktionsweise. Ebenso wie bei den vorangegangenen Ausführungsbeispielen ist vorgesehen, dass durch Niederdrücken des Dosierkopfes 40 eine Pumpeinrichtung 30 betätigt wird und Flüssigkeit in der Dosierkammer 42 des Dosierkopfs 40 fördert. Wenn dies in der durch die Fig. 13B und 13C verdeutlichten Weise geschehen ist, kann Flüssigkeit ausgetragen werden.

Fig. 13D verdeutlich diesen Zustand. Zu erkennen ist, dass in der beim Austrag genutzten Überkopflage die Flüssigkeit unter Eindruck ihrer Gewichtskraft nur bis zur Austragöffnung 60 strömt und hier eine Flüssigkeitsoberfläche 108 ausbildet, ohne dabei aus der Dosierkammer 42 auszuströmen.

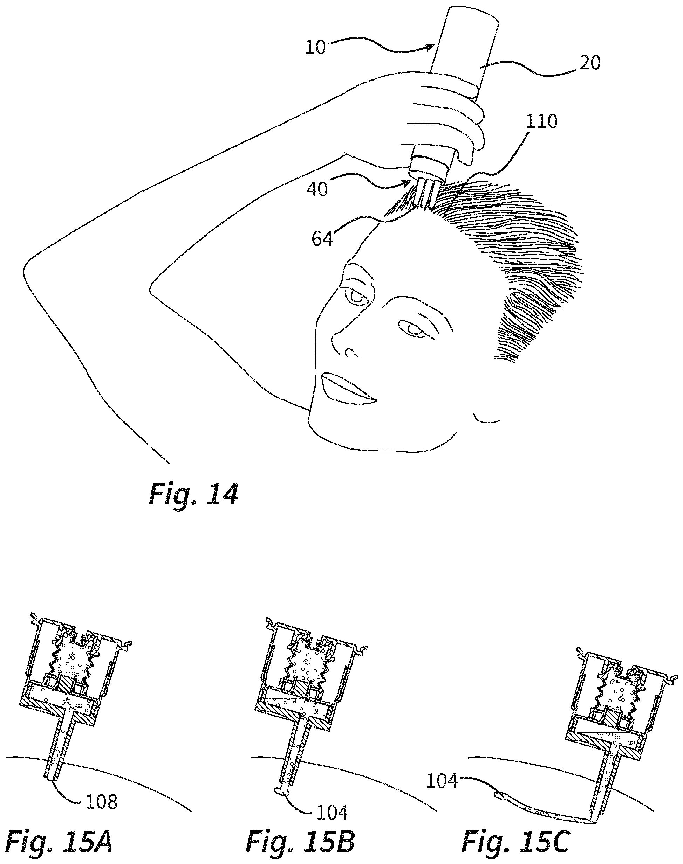

Korrespondierend mit Fig. 13D verdeutlicht Fig. 14 eine typische Anwendung eines Flüssigkeitsspenders entsprechend den Fig. 11 bis 13D. Der Flüssigkeitsspender wird in der genannten Überkopflage kammartig durch das Haar gefahren.

Wie die Fig. 15A bis 15C verdeutlichen, führt die Berührung der an der Austragöffnung 60 anstehenden Flüssigkeitsoberfläche 108 mit der Kopfoberfläche dazu, dass die Flüssigkeitsoberfläche 108 gestört wird und Flüssigkeit dann kontinuierlich entsprechend der Darstellung der Fig. 15C ausgetragen werden kann und einen Flüssigkeitsfilm 104 bildet. Wird der Flüssigkeitsspender 10 angehoben, so unterbricht der Flüssigkeitsfluss, so dass anschließend der Flüssigkeitsspender ggf. nach einer Neubefüllung der Dosierkammer 42 mittels der Pumpeinrichtung 30, neu angesetzt werden kann.

Fig. 16 zeigt eine alternative Gestaltung eines solchen Flüssigkeitsspenders gemäß der dritten Variante. Dessen Besonderheit ist, dass die Applikatorspitzen 64 nicht gradlinig sind, sondern mit einem Knick versehen. Diese Ausrichtung ermöglicht eine Applikation der Flüssigkeit auf der Haut bei verringerter Gefahr, versehentlich hierbei die Pumpeinrichtung 30 zu betätigen.

## Patentansprüche

1. Flüssigkeitsspender (10) zum dosierten Austrag von Flüssigkeit mit den folgenden Merkmalen:
a. der Flüssigkeitsspender (10) weist einen Flüssigkeitsspeicher (20) zur Lagerung der Flüssigkeit vor dem Austrag auf, und
b. der Flüssigkeitsspender (10) weist einen Dosierkopf (40) mit einer Dosierkammer (42) zur Aufnahme einer Charge der Flüssigkeit auf, der gegenüber dem Flüssigkeitsspeicher (20) verlagerbar ist, und
c. der Flüssigkeitsspender (10) weist eine Pumpeinrichtung (30) auf, die zwischen dem Flüssigkeitsspeicher (20) und der Dosierkammer (42) angeordnet ist, so dass eine Pumpkammer (32) der Pumpeinrichtung (30) durch Verlagerung des Dosierkopfes (40) in einer Pumprichtung (2) Flüssigkeit in die Dosierkammer (42) fördert,
**gekennzeichnet durch** das folgende weitere Merkmal:
d. der Flüssigkeitsspender (10) verfügt über eine die Dosierkammer (42) begrenzende Applikatorwandung (80) am Dosierkopf (40), die für den unmittelbaren Körperkontakt vorgesehen ist und die von einer Austragöffnung (82) oder einer Mehrzahl von Austragöffnungen durchdrungen ist, wobei die Applikatorwandung (80) gegenüber angrenzenden Wandungen (84) der Dosierkammer (42) als Ganzes oder durch Verformung in einer Verlagerungsrichtung (6) verlagerbar ist.

2. Flüssigkeitsspender (10) nach Anspruch 1 mit dem folgenden weiteren Merkmal:
a. die Applikatorwandung (80) ist als Ganzes in der Verlagerungsrichtung (6) verlagerbar und weist eine mit der Applikatorwandung (80) verbundene Führungsfläche (86A) auf, die abdichtend und verschiebbar an einer korrespondierenden umgebenden Führungsfläche (86B) des Dosierkopfes (40) anliegt,
vorzugsweise mit dem folgenden zusätzlichen Merkmal:
b. es ist eine Federeinrichtung vorgesehen, die die Applikatorwandung (80) in Richtung einer äußeren Endlage kraftbeaufschlagt.

3. Flüssigkeitsspender (10) nach Anspruch 1 mit dem folgenden weiteren Merkmal:
a. die Applikatorwandung (80) ist als durch elastische Verformung einer Dosierkammerwandung (88) in einer Verlagerungsrichtung (6) auslenkbare Wandung gestaltet,
insbesondere mit mindestens einem der folgenden zusätzlichen Merkmale:
b. die Applikatorwandung (80) ist in einem Ruhezustand ohne Körperkontakt nach außen gewölbt und vorzugsweise in sich elastisch verformbar, und/oder
c. die Applikatorwandung (80) ist als bistabile Applikatorwandung (80) ausgebildet, die eine erste und ein zweite stabile Form einnehmen kann, wobei die erste Form der Applikatorwandung (80) eine nach außen gewölbte Form ist, die die Applikatorwandung (80) bei befüllter Dosierkammer einnimmt, und wobei die zweite Formgebung der Applikatorwandung (80) eine nach innen gewölbte Form ist, die die Applikatorwandung (80) bei Volumenreduzierung der Dosierkammer (42) durch Andrücken an die Haut einnimmt.

4. Flüssigkeitsspender nach Anspruch 3 mit dem folgenden weiteren Merkmal:
a. die Dosierkammerwandung (88) ist zumindest abschnittsweise als ein stauchbarer Balgabschnitt (89) ausgebildet, wobei der Balgabschnitt (89) durch Andrücken der Applikatorwandung (80) an die Haut zusammendrückbar und die Dosierkammer (42) hierdurch verkleinerbar ist, so dass Flüssigkeit durch die mindestens eine Austragöffnung (82) hindurch ausströmen kann.

5. Flüssigkeitsspender (10) nach einem der vorstehenden Ansprüche mit dem folgenden weiteren Merkmal:
a. der Dosierkopf (40) weist eine Rasteinrichtung (90) auf, mittels derer die Applikatorwandung (80) nach Entleerung in einer Minimum-Stellung gehalten wird, bei der die Dosierkammer (42) ein minimales Volumen aufweist,
vorzugsweise mit mindestens einem der folgenden Merkmale:
b. die Rasteinrichtung (90) ist derart ausgebildet, dass sie durch den durch die Pumpeinrichtung (30) erzeugbaren Druck gelöst werden kann, insbesondere durch anliegenden Flüssigkeitsdruck, so dass die Dosierkammer (42) durch gegen die Rasteinrichtung drückende Flüssigkeit wieder vergrößerbar ist,
c. die Rasteinrichtung (90) weist einen Rastkörper (92) auf, der mit der Applikatorwandung (80) gemeinsam verlagerbar ist und in einer Endlage mit minimalem Volumen der Dosierkammer (42) mit einer Haltestruktur (94) des Dosierkopfes (40) zusammenwirkt, so dass die Applikatorwandung (80) in der Minimum-Stellung verbleibt, und/oder
d. die Rasteinrichtung (90) ist in einem Flüssigkeitskanal (43) der Dosierkammer (42) vorgesehen und verschließt diesen Flüssigkeitskanal (43), wenn sich die Applikatorwandung (80) in der Minimum-Stellung befindet.

6. Flüssigkeitsspender nach einem der vorstehenden Ansprüche mit dem folgenden weiteren Merkmal:
a. die Verlagerungsrichtung (6) ist nicht-parallel zur Pumprichtung (2) ausgerichtet, vorzugsweise mit mindestens einem der folgenden Merkmale:
b. ein eingeschlossener Winkel zwischen der Verlagerungsrichtung (6) und der Pumprichtung (2) beträgt vorzugsweise mindestens 30°, insbesondere vorzugsweise zwischen 45° und 75°, und/oder
c. der eingeschlossene Winkel zwischen der Verlagerungsrichtung (6) und der Pumprichtung (2) ist derart auf eine Führung (38) zwischen dem Dosierkopf (40) und dem Flüssigkeitsspeicher (20) angepasst, dass eine Verlagerung des Dosierkopfes (40) gegenüber dem Flüssigkeitsspeicher (20) in Folge einer Kraftbeaufschlagung der Applikatorwandung (80) in Verlagerungsrichtung (6) nicht eintritt.

7. Flüssigkeitsspender nach einem der vorstehenden Ansprüche mit den folgenden weiteren Merkmalen:
a. der Dosierkopf (40) weist an einer dem Flüssigkeitsspeicher (20) abgewandten Seite eine Betätigungsfläche (50) zum Niederdrücken des Dosierkopfes (40) auf, und
b. der Dosierkopf (40) weist seitlich der Betätigungsfläche (50) einen bezogen auf eine Längsachse in Pumprichtung die Betätigungsfläche (50) überragenden Applikatordom (78) auf, an dem die Applikatorwandung (80) vorgesehen ist,
vorzugsweise mit dem zusätzlichen Merkmal:
c. der Applikatordom (78) erstreckt sich bezogen auf eine Querrichtung bis über die Betätigungsfläche (50).

8. Flüssigkeitsspender (10) zum dosierten Austrag von Flüssigkeit mit den folgenden Merkmalen:
a. der Flüssigkeitsspender (10) weist einen Flüssigkeitsspeicher (20) zur Lagerung der Flüssigkeit vor dem Austrag auf, und
b. der Flüssigkeitsspender (10) weist einen Dosierkopf (40) mit einer Dosierkammer (42) zur Aufnahme einer Charge der Flüssigkeit auf, der gegenüber dem Flüssigkeitsspeicher (20) verlagerbar ist, und
c. der Flüssigkeitsspender (10) weist eine Pumpeinrichtung (30) auf, die zwischen dem Flüssigkeitsspeicher (20) und der Dosierkammer (42) angeordnet ist, so dass eine Pumpkammer (32) der Pumpeinrichtung (30) durch Verlagerung des Dosierkopfes (40) in einer Pumprichtung (2) Flüssigkeit in die Dosierkammer (42) fördert,
**gekennzeichnet durch** das folgende weitere Merkmal:
d. der Dosierkopf (40) verfügt über eine die Dosierkammer (42) begrenzende Dosierkammerwandung (70), die gegen eine Federkraft auslenkbar ist, sowie über mindestens eine Austragöffnung (60) und ein der Austragöffnung (75) zugeordnetes Schaltventil (76), welches für den unmittelbaren Körperkontakt vorgesehen ist und welches durch Kraftbeaufschlagung geöffnet werden kann, so dass Flüssigkeit aus der Dosierkammer (42) unter Entspannung der auslenkbaren Dosierkammerwandung (70) durch die Austragöffnung (60) hindurch ausströmen kann,
vorzugsweise mit dem folgenden zusätzlichen Merkmal:
e. die auslenkbare Dosierkammerwandung (70) weist eine Durchbrechung auf, durch die hindurch die Flüssigkeit zur Austragöffnung strömen kann.

9. Flüssigkeitsspender (10) nach Anspruch 8 mit den folgenden zusätzlichen Merkmalen:
a. die die Dosierkammer begrenzende Dosierkammerwandung (70) ist als elastische Dosierkammerwandung ausgestaltet, die durch Befüllung der Dosierkammer (42) mittels der Pumpeinrichtung (30) elastisch gespannt wird,
vorzugsweise mit dem zusätzlichen Merkmal:
b. der Dosierkopf weist einen innenliegenden und von außen unzugänglichen Raum (74) auf, in den die elastisch auslenkbare Dosierkammerwandung (70) durch Befüllung der Dosierkammer (42) ausgelenkt wird.

10. Flüssigkeitsspender (10) zum dosierten Austrag von Flüssigkeit mit den folgenden Merkmalen:
a. der Flüssigkeitsspender (10) weist einen Flüssigkeitsspeicher (20) zur Lagerung der Flüssigkeit vor dem Austrag auf, und
b. der Flüssigkeitsspender (10) weist einen Dosierkopf (40) mit einer Dosierkammer (42) zur Aufnahme einer Charge der Flüssigkeit auf, der gegenüber dem Flüssigkeitsspeicher (20) verlagerbar ist, und
c. der Flüssigkeitsspender (10) weist eine Pumpeinrichtung (30) auf, die zwischen dem Flüssigkeitsspeicher (20) und der Dosierkammer (42) angeordnet ist, so dass eine Pumpkammer (32) der Pumpeinrichtung (30) durch Verlagerung des Dosierkopfes (40) in einer Pumprichtung (2) Flüssigkeit in die Dosierkammer (42) fördert,
**gekennzeichnet durch** das folgende weitere Merkmal:
d. der Flüssigkeitsspender (10) verfügt über mindestens eine sich an die Dosierkammer anschließende Austragöffnung (60) am Dosierkopf (40) zum Austrag von Flüssigkeit, die derart gestaltet und mit der Dosierkammer (42) verbunden ist, dass aus der Dosierkammer (42) nach unten in Richtung der Austragöffnung (60) strömende Flüssigkeit durch die Oberflächenspannung der Flüssigkeit (100) an der Austragöffnung (60) verbleibt, bis die Oberflächenspannung durch Kontakt mit einer Körperoberfläche (110) unterbrochen wird,
vorzugsweise mit dem zusätzlichen Merkmal:
e. der Flüssigkeitsspender weist eine Mehrzahl von Austragöffnungen (60) auf, wobei die Austragöffnungen (60) derart gestaltet und mit der Dosierkammer (42) verbunden sind, dass auch dann, wenn durch eine erste der Austragöffnungen Luft frei in die Dosierkammer strömen kann, aus der Dosierkammer (42) nach unten in Richtung einer zweiten der Austragöffnungen (60) strömende Flüssigkeit durch die Oberflächenspannung der Flüssigkeit (100) an der Austragöffnung (60) verbleibt, bis die Oberflächenspannung durch Kontakt mit einer Körperoberfläche (110) unterbrochen wird.

11. Flüssigkeitsspender (10) nach Anspruch 10 mit den zusätzlichen Merkmalen:
a. der Dosierkopf (40) weist mindestens eine schlanke Applikatorspitze (64) auf, an deren distalem Ende die mindestens eine Austragöffnung (60) vorgesehen ist,
vorzugsweise mit mindestens einem der zusätzlichen Merkmale:
b. der Dosierkopf (40) weist eine Mehrzahl von Applikatorspitzen (64) auf, die parallel zueinander ausgerichtet sind und an deren Ende jeweils eine Austragöffnung (60) vorgesehen ist, und/oder
c. die Länge der Applikatorspitze beträgt zwischen 5 mm und 20 mm, vorzugsweise mindestens 10 mm und/oder höchstens 15 mm, und/oder
d. mindestens eine Applikatorspitze weist eine Haupterstreckungsrichtung auf, die mit der Pumprichtung übereinstimmt und/oder
e. mindestens eine Applikatorspitze weist eine Haupterstreckungsrichtung und/oder eine Erstreckungsrichtung im Bereich der Austragöffnung auf, die gegenüber der Pumprichtung angewinkelt ist, vorzugsweise um einen Winkel von mindestens 30°, insbesondere vorzugsweise um mehr als 60°.

12. Flüssigkeitsspender (10) nach einem der Ansprüche 10 oder 11 mit einem der zusätzlichen Merkmale:
a. der Dosierkopf (40) weist an einer dem Flüssigkeitsspeicher (20) abgewandten Seite eine Betätigungsfläche (50) zum Niederdrücken des Dosierkopfes (40) auf, wobei die mindestens eine Austragöffnung (60), insbesondere die mindestens eine Applikatorspitze (64) oder die Gesamtheit der Applikatorspitzen (64) neben der Betätigungsfläche (50) und exzentrisch zu einer Mittelachse (8) des Dosierkopfes vorgesehen ist, oder
b. der Dosierkopf weist an einer dem Flüssigkeitsspeicher abgewandten Seite eine Betätigungsfläche mit zwei Betätigungsteilflächen auf, wobei mindestens eine Austragöffnung, insbesondere mindestens eine Applikatorspitze mit daran vorgesehener Austragöffnung, zwischen den beiden Betätigungsteilflächen vorgesehen ist.

13. Flüssigkeitsspender (10) nach einem der Ansprüche 10 bis 12 mit mindestens einem der zusätzlichen Merkmale:
a. die mindestens eine Austragöffnung (60) ist kreisrund, und/oder
b. die mindestens eine Austragöffnung (60) weist einen lichten Querschnitt von maximal 3 mm auf, und/oder
c. die mindestens eine Austragöffnung (60) ist von einem ringförmigen Anhaftbereich umgeben, dessen Breite mindestens 1 mm beträgt.

14. Flüssigkeitsspender (10) nach einem der vorstehenden Ansprüche mit mindestens einem der zusätzlichen Merkmale:
a. die Dosierkammer (42) weist ein Innenvolumen zwischen 0,1 ml und 10 ml auf und/oder
b. die Pumpeinrichtung ist derart ausgebildet, dass zur Verlagerung des Dosierkopfes in Richtung des Flüssigkeitsspeichers eine Kraft von mindestens 10 Newton, vorzugsweise von mindestens 15 Newton, erforderlich ist, und/oder
c. ein maximales Fördervolumen der Pumpeinrichtung (30) bei einer Pumpbetätigung beträgt zwischen 20% und 90% des Volumens der Dosierkammer (42), insbesondere zwischen 20% und 40% des Volumens der Dosierkammer (42).

15. Flüssigkeitsspender nach einem der vorstehenden Ansprüche mit den weiteren Merkmalen:
a. der Flüssigkeitsspeicher (20) ist mit einer Flüssigkeit zur Behandlung der Kopfhaut befüllt, insbesondere mit einem Minoxidil-Präparat, oder
b. der Flüssigkeitsspeicher (20) ist mit einer Hautcreme befüllt.
